(19) Europäisches Patentamt
European Patent Office
Office européen des brevets

(11) Publication number: **0 163 490**
**A1**

(12) # EUROPEAN PATENT APPLICATION

(21) Application number: 85303624.2

(22) Date of filing: 22.05.85

(51) Int. Cl.[4]: A 61 K 7/06
A 61 K 31/57
//(A61K31/57, 31:565)

(30) Priority: 22.05.84 GB 8413070

(43) Date of publication of application: 04.12.85 Bulletin 85/49

(84) Designated Contracting States: AT BE CH DE FR GB IT LI LU NL SE

(71) Applicant: Mortimer, Christopher Harry, Dr.
8 Park Square West Regents Park
London NW1 4LJ(GB)

(72) Inventor: Mortimer, Christopher Harry, Dr.
8 Park Square West Regents Park
London NW1 4LJ(GB)

(74) Representative: Baverstock, Michael George Douglas et al,
BOULT, WADE & TENNANT 27 Furnival Street
London, EC4A 1PQ(GB)

(54) **Treatment of female hair loss.**

(57) A pharmaceutical formulation for use in treating female scalp hair loss which comprises cyproterone acetate (a metabolite, analogue or derivative thereof) and at least one oestrogen, wherein when prepared in unit dosage form the formulation is adapted to administer at least 125mg and not more than 750mg of cyproterone acetate per month.

EP 0 163 490 A1

Croydon Printing Company Ltd.

# TREATMENT OF FEMALE HAIR LOSS

The present invention relates to the treatment of scalp hair loss in women. This is a distressing condition which can affect women from puberty and which without treatment generally progressively worsens, albeit with periods in which the patients' condition remains stable. Because thinning hair is not a socially-acceptable condition in women, as it may be in men after a certain age, the condition can cause, not merely distress, but severe psychological symptoms in women involving feelings of loss of femininity and self confidence, depression an inability to concentrate on anything except the prospect of impending baldness with mounting embarassment.

The present invention provides a pharmaceutical formulation for use in treating female scalp hair loss which comprises cyproterone acetate (a metabolite, analogue or derivative thereof) and at least one oestrogen.

Cyproterone acetate is an anti-androgenic compound of the formula :-

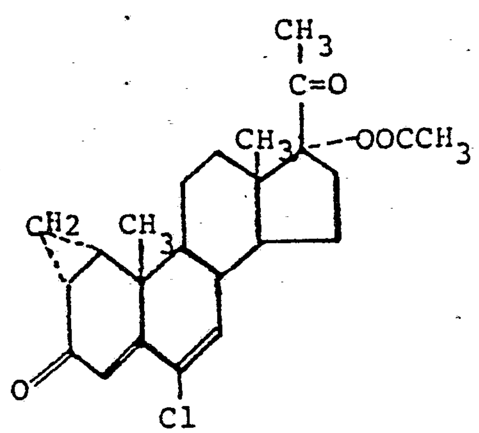

(17 alpha - Acetoxy -6- chloro - 1 alpha, 2 alpha - methylene-pregna - 4, 6 diene-3, 20 - dione acetate - conveniently referred to as "CPA".) It has been used previously in the treatment of men for control of libido in severe hypersexuality and has been proposed

for use in treating precocious puberty.

In addition, CPA has been used in combination with ethinyloestradiol (EE), in clinical trials in women for the treatment of acne, hitsutism and virilism, as described in J. Hammerstein et al J. Steroid Biochem.1975. Vol 6 pp 827-836. The dose levels used in those trials were, however, adjusted in accordance with the patients' responses in respect of acne, hirsutism and virilism. The predominant dosage used was 100mg CPA given daily from day 5 until day 14, together with 50ug EE administered daily from day 5 until day 25 of the patient's cycle. The Hammerstein Article says that "only rarely, it may become feasible to increase the daily CPA dosage up to 200mg or to decrease the dosage to 50mg, but in general 100mg seems just the correct daily dose". Very mild cases of these disorders have been described as having been treated with 2mg CPA + 50ug EE from day 5 until day 25 of the patient's cycle.

A contraceptive having this composition was found to be largely ineffective in the treatment of moderate and severe acne vulgaris when taken over a period of six months (Mugglestone C.J. Rhodes E.L. Clin. Exp. Dermatol. 1982. 7: 593-598).

In the Article of Hammerstein et al reference is made to the effect of a CPA - EE combination treatment on alopecia (hair loss) in women with androgen over-production. A failure rate of 40 - 50% was reported after one year of therapy, although no numerical data are presented as to how the response to treatment was determined. A suggestion was made that the poor absorption rate of CPA was responsible for this lack of success. It was further suggested that researchers should seek "better balanced

preparations with no depot properties", but no guidance is given in the Article to how this "balance" may be achieved. Dawber et al. Br. J. Dermatol 1982: Vol. 107, Suppl 20, reported that 2mg CPA + 50ug EE daily for 21 consecutive days proved ineffective in maintaining existing hair growth in women with androgenic alopecia. No further advances have apparently been made in this field since these Articles were published and the condition of androgenic alopecia in women is generally thought to be untreatable.

It has now been found unexpectedly that not only can the process of androgenic alopecia in women be arrested, but also new hair growth can be stimulated, by the use of CPA at a dose level which exceeds a certain "threshold" value. Naturally, this threshold varies from patient to patient, but it has generally been found to be a monthly total of at least 125mg, and preferably 500mg. Dosages higher than 750mg oer month usually produce unwanted side effects and are not significantly more efficacious than the preferred dosage.

In accordance with the present invention there is provided a pharmaceutical formulation for use in treating female scalp hair loss which comprises cyproterone acetate (a metabolite, analogue or derivative thereof) and at least one oestrogen, wherein when prepared in unit dosage form the formulation is adapted to administer at least 125mg and not more than 750mg of cyproterone acetate per month.

The invention further provides, in combination, a pharmaceutical formulation of the invention, a container therefor, and instructions for the use of the pharmaceutical formulation in the treatment of

female scalp hair loss.

The formulations of the present invention may be designed for oral or parenteral administration. Although the formulations could be for administration by injection, they will most conveniently be for oral administration, e.g. as tablets, capsules or solutions or suspensions.

They may however be formulated as creams or lotions for topical administration or as injectable solutions or suspensions for subcutaneous injection, e.g. in the scalp, or for other forms of injection. They may generally be formulated for any of the known routes of pharmaceutical administration.

Preferably, a formulation for oral administration will contain from 10 to 75mg, preferably about 50mg, of cyproterone acetate per unit dosage form, e.g. per tablet.

The identity of the oestrogen used is not critical. Ethinyloestradiol is preferred. The oestrogen is present to provide a contraceptive effect since administration of cyproterone acetate during pregnancy would be highly undesirable. In addition, oestrogen has the beneficial effect or raising plasma sex hormone binding globulin (SHBG) levels thereby diminishing free (biologically-active) testosterone and other androgen levels. A suitable dose rate for ethinyloestradiol would be 20 to 60µg per day orally, preferably about 40µg.

The treatment of female scalp hair loss will preferably involve the administration of cyproterone acetate and the oestrogen in a cyclic manner. In the first part of cycle preferably both cyproterone acetate and oestrogen are administered. This is followed preferably by treatment with oestrogen alone

and then by no treatment to allow withdrawal bleeding. Preferably the cycle length is 28 days in accordance with normal contraceptive practice.

A possible oral dosage regimen would therefore be:
Ethinyloestradiol 20 to 60 µg daily for 28 days of each cycle
Cyproterone acetate 10 to 75 mg daily for up to 27 days of the cycle;
or more preferably :-
Ethinyloestradiol at 30 - 40 µg daily for the first 21 days of each cycle, and
Cyproterone acetate at about 50 mg daily for the first 10 days of each cycle.
The dosages most suitable for oestrogens other than ethinyloestradiol will be well known to practioners.

Whilst the dosages of oestrogen and cyproterone acetate for administration on the same day may conveniently be formulated together as a formulation according to the invention, they can equally well be taken separately.

In either case, it will be advantageous if the two different types of daily dose, namely oestrogen pluc cyproterone acetate or oestrogen alone are put up in a calendar pack as is the normal practice with contraceptive tablets. Optionally, tablets containing neither active ingredient (dummy tablets) may be provided for the days when neither ingredient is to be taken.

Accordingly, the present invention provides a calendar pack containing pharmaceutical formulation doses for use in treating female scalp hair loss, comprising spaced locations corresponding to days of a menstrual cycle, dosage forms in a first series of said locations providing at each location of the series a daily dose of cyproterone acetate and a daily dose of an oestrogen in pharmaceutically administerable form and dosage forms at a second

series of locations following the first series providing a daily dose of the oestrogen in pharmaceutically administerable form without cyproterone acetate, wherein the total dosage of cyproterone acetate in the pack is at least 125 mg and not more than 750 mg per cycle.

An example of such a pack would be a bubble pack of the conventional kind having a tablet of other dosage form contained in a bubble at each of a series of spaced locations, normally positioned in a closed loop running around the edge of the pack. The locations may be numbered, normally 1 to 28.

A first series of bubbles may contain either one tablet or other dosage form containing both active ingredients or a pair of tablets or other dosage forms, one containing oestrogen and the other cyproterone acetate. Where two dosage forms are provided they may of course be put in separate bubbles at the same day's location. The first series of bubbles will preferably correspond to up to the first 27 days, e.g. the first 10 days of a 28 day cycle.

The second series of bubbles may then contain only one dosage form per location containing oestrogen but not cyproterone acetate. The second series may comprise enough locations to bring the user to about the 21st day of a cycle.

If further bubbles are provided, they may be empty so that they can simply be burst in turn to mark a day's passing or may contain a dosage form containing neither active ingredient.

An example of a tablet formulation according to the present invention would, for instance, be:-

40µg ethinyloestradiol      70.0mg lactose

| | |
|---|---|
| 50mg cyproterone acetate | 94.0mg corn starch |
| 10mg talcum | 3.2mg gelatin |
| | 2.8mg magnesium stearate |

It has been found that treatment as described above results in decreased hair fall, increased hair density, an increase in hairs of more than 40um in diameter in the frontal area of the scalp and occipital area also, as well as a decrease in the greasiness of the hair. Various courses of treatment and their effects are described in the following clinical examples in which reference is made to the accompanying drawings. In these drawings:-

Figure 1 shows frontal area trichogram changes in an untreated control group of women and a group receiving treatment. Total hair density (THD) (normal range 234-345 hairs/$cm^2$) is indicated by open bars; meaningful hair density (MHD) (normal range 193-290 hairs greater than 40um in diameter/$cm^2$) by vertical lined bars, with the Mean $\pm$ s.e. being shown.

Figures 2a and 2b show frontal area trichogram changes in total hair density (THD) and meaningful hair density (MHD), respectively, during treatment expressed as a percentage improvement on basal values. (The mean line is also shown)

Figure 3 shows occipital area trichogram changes in the control and treatment groups as in Figure 1. Normal total hair density (THD) is 233-352 hairs/$cm^2$ and meaningful hair density (MHD) 198-310 hairs greater than 40µm in diameter/$cm^2$.

Figures 4a and 4b show occipital area trichogram changes as in Figures 2a and 2b, respectively.

Figure 5 shows the effect of dose variation on the frontal area trichogram after 12 months of cyproterone acetate (CPA) 500mg/month during treatment. Total hair density (THD) is indicated by open bars, meaningful hair density (MHD) by vertical lined bars. Patient aged 26 years with hair loss for 2 years prior to treatment. (No family history).

Figure 6 shows the effect of dose variation on the frontal area trichogram after 24 months of cyproterone acetate (CPA) 500mg/month during treatment. Total hair density (THD) is indicated by open bars; meaningful hair density by vertical lined bars. Patient aged 36 years with hair loss for 23 years prior to treatment. (Family history).

It has been found that it is possible that patients treated with cyclical anti-androgen therapy administered systemically as described may unexpectedly benefit further from the addition of topical preparations applied directly to the scalp.

Therefore, it is proposed that the addition of topical preparations as described may enhance the effects of systemic preparations leading to the possible reduction in the dose of either the topical or systemic preparations to produce a synergistic therapeutic effect. For this reason it may be possible for the lower limits of the doses described previously to be reduced while maintaining the effectiveness of the preparations (systemic and topical) in the arrest and reversal of the common baldness process. Such a combination of therapy

would be of considerable practical clinical value in allowing the minimum effective dose of treatment to be used.

CLINICAL EXAMPLES

Thirteen women aged between 18-37 years with a history of common baldness (diffuse androgenic alopecia/genetic hair loss) for 2-23 years and continued hair loss were treated with cyclical antiandrogen therapy (CAT) for up to 30 months.

Seven women aged between 24 and 43 years with a history of scalp hair loss for 6 to 18 years and a family history in 3 acted as controls and remained untreated for six months. Three of them, one with a family history, subsequently entered the treatment group.

Their data at the end of six months control period are included in the time 0 grouped results. One patient was treated for six months and two for twelve months.

The treatment group received CAT comprising cyproterone acetate 50mg daily for 10 days following the period combined with ethinyl oestradiol 40µg daily for 21 days following the period repeated cyclically allowing 5-7 days for withdrawal bleeds. Two patients aged 26 and 36 years and a history of hair loss of 2 and 23 years, with a family history in the latter, received the standard treatment regimen for 12 months in the case of the younger patient (Fig. 5) and for 24 months in the elder patient (Fig 6). At this stage the total amount of cyproterone acetate given in equal doses over 10 days/month was reduced from 500mg (i.e. 10 x 50mg) to 250mg (i.e. 10 x 25mg) or 125mg (i.e. 5 x 25mg alternate days) per month. The dose of ethinyloestradiol (840µg/month) was unchanged. The ingredients were taken individually or combined in powdered form in gelatin capsules.

Total hair density (THD). individual fibre diameter and meaningful hair density (MHD) (hairs greater than 40μm in diameter/$cm^2$) were compared with values obtained previously in 10 normal women in whom unit area trichograms had been performed. The assessment of androgen -dependent alopecia using the unit area trichogram technique is described in general in Rushton H., James K.C., Mortimer C.H.; British Journal of Dermatology, (1983) 109, 429-437.

All trichograms were performed following the same standardised procedure prior to sampling. This involved washing the hair in the morning of the first day (day 1), followed by combing the hair four times (morning, midday, afternoon and evening) The combing regimen was repeated on day 2. On day 3, the hair was combed in the morning and 2 hours later the hair samples were taken.

Hairs were plucked from the frontal area 10-30mm right of the midline and 35-55mm from the frontal periphery. The occipital site was plucked 0-30mm to the right of the midline and 70-110mm from the occipital periphery, while in two patients the vertex area was sampled also. For each trichogram the midline was defined as that extending from the nose to the occipital protuberance. The precise dimensions of the plucked area were determined by Macro 1:1 photography using a Canon Fln camera. The frontal, occipital and vertex area was defined by marking the scalp through a rigid template with a felt tipped pen. All the hairs within and on the marked line were removed singly in the direction of hair growth. The frontal area sampled varied between 32-38$mm^2$ due to the type of felt tipped pen employed.

Repeated measurement of the same sites by two independent observers produced a maximum variation in area recorded of less than 8%. There was no observer difference between the total number of hairs plucked and thus the density measurement of the technique is considered to be accurate to within 8%. The unit area trichogram was performed before and after six months without treatment in the control group and at six monthly intervals during CAT.

In the control group of seven patients total frontal hair density basally ranged between 69-206, mean 149 ± s.e. 18 hairs/$cm^2$ (normal range 234-345 hairs/$cm^2$) and showed no significant change after six months, 65-94, mean 139 ± s.e. 16 hairs/$cm^2$. Meaningful hair density also showed no significant change from 58-167, mean 111 ± s.e. 14 hairs/$cm^2$ basally (normal range 193-290 hairs greater than 40μm in diameter/$cm^2$) to 65-162, mean 108 ± s.e. 12 hairs/$cm^2$ after six months. In contrast, during CAT there was an increase in both total hair and meaningful hair density. All eight patients who were measured at six months showed a significant increase ($P<0.01$) in total hair density from 98-194, mean 142 ± s.e. 10 hairs/$cm^2$ basally to 132-264 mean 173 ± s.e. 13 hairs/$cm^2$, an increase in total hair density of 4-36% on basal values at six months. After 12 months there was a further increase in total hair density in all 13 patients from basal values of 98-218, mean 146 ± s.e. 9 to 173-280, mean 197 ± s.e. 9 hairs/$cm^2$ ($P<0.001$) and to 168-244, mean 202 ± s.e. 11 hairs/$cm^2$ after 18 months of treatment ($P<0.01$).

This represented an increase in total hair density in the group between 26-71% on basal total hair density during CAT. Basal meaningful hair density ranged between 65-182, mean 103 ± s.e. 9 hairs/$cm^2$. After six months of treatment five of the eight patients showed a significant increase ($P < 0.02$) in meaningful hair density from, 65-162, mean 99 ± s.e. 9 basally to 91-193, mean 122 ± s.e. 13 hairs/$cm^2$. After 12 months there was a clear increase in meaningful hair density in all 13 patients to 105-227, mean 145 ± s.e. 11 hairs/$cm^2$ ($P < 0.001$) and 105-208, mean 143 ± s.e. 12 hairs/$cm^2$ after 18 months ($P < 0.02$). This represented an increase in meaningful hair density between 15-92% on basal values during CAT. These results are shown in Figures 1, 2a, 2b.

In the control group of seven patients, five had occipital area trichograms performed. Total hair density basally ranged between 159-211, mean 184 ± s.e. 9 hairs/$cm^2$ (normal range 233-352 hairs/$cm^2$) and showed no significant change after six months 139-200, mean 174 ± s.e. 10. Meaningful hair density also showed no significant change from 113-160, mean 143 ± s.e. 9 hairs/$cm^2$ basally (normal range 198-310 hairs greater than 40μm in diameter/$cm^2$) to 107-167, mean 137 ± s.e. 10 hairs/$cm^2$ at six months. During CAT four patients showed an increase in total hair density, from 144-264 basally to 196-283 hairs/$cm^2$ at 6 months, while in one there was no significant difference at 6 or 12 months. The remaining 9 patients at 12 months had increased total hair density from 141-276 basally mean 188 ± s.e. 12 hairs/$cm^2$ to 178-293, mean 222 ± s.e. 13 hairs/$cm^2$.

After 18 months the previously unresponsive patient had an increase in total hair density from 183 basally to 222 hairs/$cm^2$. These changes represented a significant increase ($P < 0.01$) in total hair density in the group and a 21-56% improvement over basal values during CAT. Three patients showed a marked increase in meaningful hair density from 118-221 basally to 152-233 hairs/$cm^2$ at six months, although two remained unchanged. After 12 months one patient (as with total hair density) showed no significant change. However, the remaining 9 patients had a significant increase in meaningful hair density from 113-221 hairs/$cm^2$ basally, mean 145 $\pm$ s.e. 10 hairs/$cm^2$ to 132-233, mean 182 $\pm$ s.e. 12 hairs/$cm^2$ ($P < 0.001$). After 18 months the previously unresponsive patient had an increase in meaningful hair density from 113 basally to 144 hairs/$cm^2$. The changes in the group as a whole represented an increase in meaningful hair density between 10-54% on basal values during CAT. These results are summarised in Figures 3, 4a, 4b.

In two patients, the most severely affected area of hair loss was at the vertex, and hair density changes were followed during CAT over 12-18 months. Both patients showed an increase in total hair density at the vertex from 111 to 144 basally to 124 or 148 at 12 months with a further increase to 200 hairs/$cm^2$ in the latter at 18 months. Meaningful hair density also increased, from 73 or 114 to 124 or 137 at 12 months and to 173 hairs/$cm^2$ in the latter at 18 months. Normal data for the vertex area are not yet available.

Two women who achieved increases in both frontal total hair density and meaningful hair density at 6 and 12 months while receiving the standard treatment regimen were selected to assess the effects of dose variation.

After twelve months, the cyproterone acetate dose in one patient was reduced from 500mg/month (standard) to 250mg/month for a further twelve months. Total hair density continued to increase from 147, basally, to 202 hairs/$cm^2$, while the meaningful hair density improved from 111, basally to 173 hairs/$cm^2$ at 24 months. However, when the total dose of cyproterone acetate was further reduced to 125mg/month over the next six months the patient noted an increase in hair fall within three months and there was a reduction in total hair density from 202 to 171 hairs/$cm^2$ and meaningful hair density from 173 to 131 hairs/$cm^2$. The decrease in hair density was clinically obvious. These results are shown in Fig. 5. In the second patient the standard treatment regimen was continued for two years during which time total hair density increased from 98, basally, to 168 hairs/$cm^2$ and meaningful hair density from 65, basally, to 125 hairs/$cm^2$. When the dose of cyproterone acetate was reduced to 250mg/month for 6 months total hair density fell from 168 to 139 hairs/$cm^2$ and the meaningful hair density from 125 to 113 hairs/$cm^2$. The patient herself was uncertain as to whether there had been a deterioration although this had been suggested by her mother. This was the impression clinically. These results are shown in Fig. 6.

During treatment all patients remarked on the reduction of scalp and hair greasiness within 3-6 weeks (except one patient (Fig. 6) who had a consistently normal-dry scalp). All noted a reduction in hair fall during combing or washing after 2-3 months. The new hair was of the same colour as that of the patients existing hair. The treatment combination proved to be contraceptive with no pregnancies recorded.

Withdrawal bleeds ocurred regularly including six patients with previously irregular cycles. Mild facial acne cleared in 3 patients by 3-8 weeks, although mild facial hirsuties remained unchanged in one patient after 18 months. One patient (Indian) noted a slight increase in skin pigmentation around the cheeks similar to chloasma. After stopping CAT in order to become pregnant, the pigmentation faded, only to reappear 6 months later when the patient was 6 weeks pregnant. All patients reported a general improvement in confidence as the increase in hair density became clinically observable.

One patient (Fig. 5), with severe depression, agoraphobia and anxiety associated with her hair loss prior to therapy, recovered completely without the need for psychotropic drugs. All patients at some stage noted an increase in breast size, although this was persistent and occasionally painful in only one patient. The breasts were otherwise normal. Weight increase was recorded in 5 patients (2-3.5kg) unchanged in 7 and reduced in one by 3.5kg. Blood pressure was not significantly changed and cervical smears after 12-24 months of treatment remained normal. There was no significant change in haematology, liver function or renal function tests.

Further clinical details relating to these studies will be reported in part, including endocrine analyses in Mortimer C.H., Rushton H., James K.C. Clin. Exp. Dermatol 1984, 9, 342-350

Statistical levels of significance were determined by the use of Students t test and by Sandler's A statistic.

3. Additional information regarding an unexpected beneficial effect upon systemically administered Cyproterone Acetate/Oestrogen by the application of topical preparations:

It has been found that it is possible that patients treated with cyclical anti-androgen therapy administered systemically as described in the Invention may unexpectedly benefit further from the addition of topical preparations applied directly to the scalp. The following topical formulations may be used in women in combination with the Invention as previously described in a volume of 1-10ml in divided doses to the affected sites on the scalp.

1) Preparation 1:

One or more of:

Oestradiol benzoate 0.2% (range 0.001-5%)

Medroxyprogesterone Acetate 0.2% (range 0.001-5%)

3,3-5 Triiodo-L-Thyronine Free Acid 20ug/3ml of solution up to 1%.

Or metabolites or derivatives or analogues thereof.

2) Preparation 1 plus vasodilator:

One or more of:

Oestradiol Benzoate 0.1% (range 0.001-5%)

Medroxyprogesterone Acetate 0.1% (range 0.001-5%)

3,3-5 Triiodo-L-Thyronine Free Acid 20ug/3ml of solution up to 1%.

Plus a vasodilator e.g:

a) Phentolamine Mesylate (as Rogitine, CIBA) 0.1% (range 0.001-10%)
b) Isoprenaline Hydrochloride 0.04% (range 0.001-10%)
c) Minoxidil 0.1%-4% (range 0.001-10%)

Or metabolites or derivatives or analogues thereof.

The addition of one or more vasodilators applied topically or given systemically could be included in the Invention. The nature of the vasodilators is not critical.

3) Preparation 2 plus "2nd Messenger"

One or more of:

Oestradiol Benzoate 0.2% (range 0.001-5%)

Medroxyprogesterone Acetate 0.2% (range 0.001-5%)

3,5 Triiodo-L-Thyronine Free Acid 20ug/3ml solution up to 1%

Vasodilator as described in 2, a,b and/or c above.

"2nd Messenger" as:

One or more of:

Cyclic AMP Free Acid 0.1% (range 0.00000000001-20%)

Cyclic AMP Sodium 0.05% (range 0.00000000001-20%)

N6, 2-O-Dibutyryl Adenosine 3,5,Cyclic Monophosphate (a long acting synthetic form of Cyclic AMP) 0.01% (range 0.00000000001-20%)

ATP Magnesium 0.1% (range 0.00000000001-20%)

Choline Theophyllinate (phosphodiesterase inhibitor) 0.2% (range 0.0001-20%)

Caffeine (phosphodiesterase inhibitor)0.2% (range 0.0001-20%)

Other "2nd Messengers" may be included according to the Invention e.g.

One or more of:

Cyclic GMP Free Acid 0.01% (range 0.00000000001-20%)

Cyclic GMP Sodium 0.1% (range 0.00000000001-20%)

N2,2-O-Dibutyryl Guanosine 3,5, Cyclic Monophosphate (a long acting synthetic form of cyclic GMP) 0.01% (range 0.00000000001-20%)

5,GDP

5,GTP

5,G Tetra P

5,Guanylyl Imidodiphosphate (a long acting form of GTP)

Inosine 3,5, Cyclic Monophosphate, Diphosphate, Triphosphate

Thymidine 3,5, Cyclic Monophosphate, Diphosphate, Triphosphate

Uridine 3,5, Monophosphate, Diphosphate, Triphosphate

Or other "2nd Messenger" systems, derivatives or analogues thereof at a dose range of 0.0000000000.1-20% or phosphodiesterase inhibitors.

4) <u>Preparation 1,2, or 3 plus enzymes of the Embden Meyerhoff Parnass Pathway, the Pentose Phosphate Shunt or the Tricarboxylic Acid cycle to include:</u>

One or more of:

| | |
|---|---|
| Phosphorylase 625 IU/L | (range 1-100,000) |
| Hexokinase 1,000 IU/L | (range 1-100,000) |
| Glucose-6-Phosphate Dehydrogenase 1,000 IU/L | (range 1-100,000) |
| Phosphofructokinase 1,000 IU/L | (range 1-100,000) |

Or other enzyme systems at a dose range of 1-100,000 IU/L.

5) <u>Preparations 1,2,3 or 4 plus:</u>

Para Amino Benzoic Acid or salts or derivatives thereof 0.1% to 0.3% (range 0.0001-20%)

6) <u>Preparations 1,2,3,4 or 5 plus:</u>

One or more other anti-androgens e.g.

| | |
|---|---|
| Spironolactone 0.1-3% | (range 0.001-20%) |
| Deoxycorticosterone 0.2% | (range 0.001-20%) |
| Cimetidine 0.1% | (range 0.001-20%) |
| Desogestrel 0.1% | (range 0.001-20%) |
| Megestrol Acetate 0.1% | (range 0.001-20%) |
| Ethynodiol Diacetate 0.1% | (range 0.001-20%) |
| Cyproterone Acetate 0.1% | (range 0.001-20%) |

| | |
|---|---|
| Lynoestrenol 0.1% | (range 0.001-20%) |
| Norethisterone 0.1% | (range 0.001-20%) |
| Levonorgestrel 0.1% | (range 0.001-20%) |
| Flutamide 0.1% | (range 0.001-20%) |
| Progesterone 0.1% | (range 0.0001-20%) |
| Azelaic Acid 0.1% | (range 0.001-20%) |
| Testolactone 0.1% | (range 0.001-20%) |
| Danazol 0.1% | (range 0.001-20%) |

Or other anti-androgens or metabolites or derivatives or analogues thereof.

7) Preparations 1,2,3,4,5 or 6 plus:

One or more of:

Immunosupressives

Amino Acids such as those normally found in the hair

Glucose or other energy source.

When the topical preparations as set out above (some components of which may be given systemically e.g. Triiodothyronine as the sodium salt given orally in the range of 1ug-300ug daily, more preferable 20ug three times daily) are administered in addition to the systemic cyclical anti-androgen therapy as described in the Invention there has resulted a clear increase in the rate of growth of scalp hair. Typically patients have reported that prior to the addition of the topical preparations they would normally cut their hair each 2-3 months to maintain the same style. However, the addition of the above preparations has resulted in hair being required to be cut each 4-6 weeks to maintain the same hair style. The rate of growth is increased particularly in the temporal and occipital areas with an increase in rate of growth in these areas being noted before that in the frontal and vertex regions. Typically the rate of hair growth has increased by approximately 25-200% during the first 3-6 months of treatment.

CLAIMS:

1. A pharmaceutical formulation for use in treating female scalp hair loss which comprises cyproterone acetate (a metabolite, analogue or derivative thereof) and at least one oestrogen, wherein when prepared in unit dosage form the formulation is adapted to administer at least 125mg and not more than 750mg of cyproterone acetate per month.

2. A formulation as claimed in claim 1 further comprising a pharmaceutically-acceptable carrier, diluent or excipient.

3. A formulation as claimed in claim 1 or claim 2 in the form of a unit dosage form for oral administration.

4. A formulation as claimed in claim 3 which provides from 10 to 75mg of cyproterone acetate per unit dosage form.

5. A formulation as claimed in any preceding claim wherein the or each oestrogen present is ethinyloestradiol or its pharmacologically-active equivalent.

6. A formulation as claimed in claim 5 when appendant to any either claim 3 or claim 4 wherein the formulation provides from 20 to 60ug per unit dosage form of ethinyloestradiol.

7. A calendar pack containing pharmaceutical formulation doses for use in treating female scalp

hair loss, comprising spaced locations corresponding to days of a menstrual cycle, dosage forms in a first series of said locations providing at each location of the series a daily dose of cyproterone acetate and a daily dose of an oestrogen in pharmaceutically-administerable form, and dosage forms at a second series of locations following the first series providing a daily dose of the oestrogen in pharmaceutically-administerable form without cyproterone acetate, wherein the total dosage of cyproterone acetate in the pack is at least 125mg and not more than 750mg per cycle.

8. A pack as claimed in claim 12 wherein the oestrogen is ethinyloestradiol or its pharmacologically-active equivalent.

9. In combination, a pharmaceutical formulation as claimed in any one of claims 1 to 6, preferably together with a topical treatment for the stimulation of hair growth for promoting female scalp hair growth., a container therefor, and instructions for the use of the pharmaceutical formulation in the treatment of female scalp hair loss.

10 The use of a combination formulation as claimed in any one of claims 1 to 6 preferably together with a topical treatment for the stimulation of hair growth for promoting female scalp hair growth.

FIG. 1.

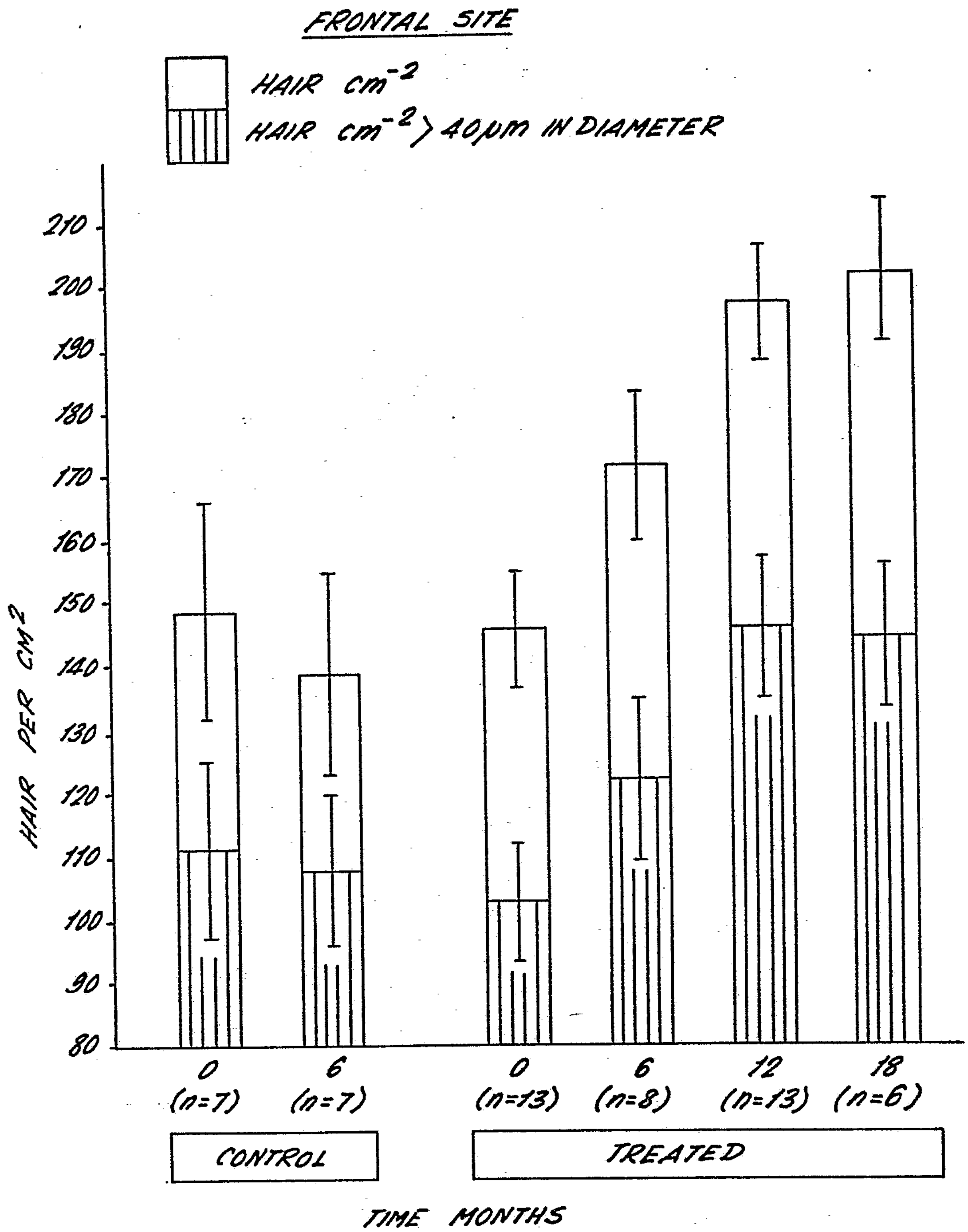
FRONTAL SITE
HAIR cm⁻²
HAIR cm⁻² > 40μm IN DIAMETER
HAIR PER CM²
210
200
190
180
170
160
150
140
130
120
110
100
90
80
0 (n=7)
6 (n=7)
0 (n=13)
6 (n=8)
12 (n=13)
18 (n=6)
CONTROL
TREATED
TIME MONTHS

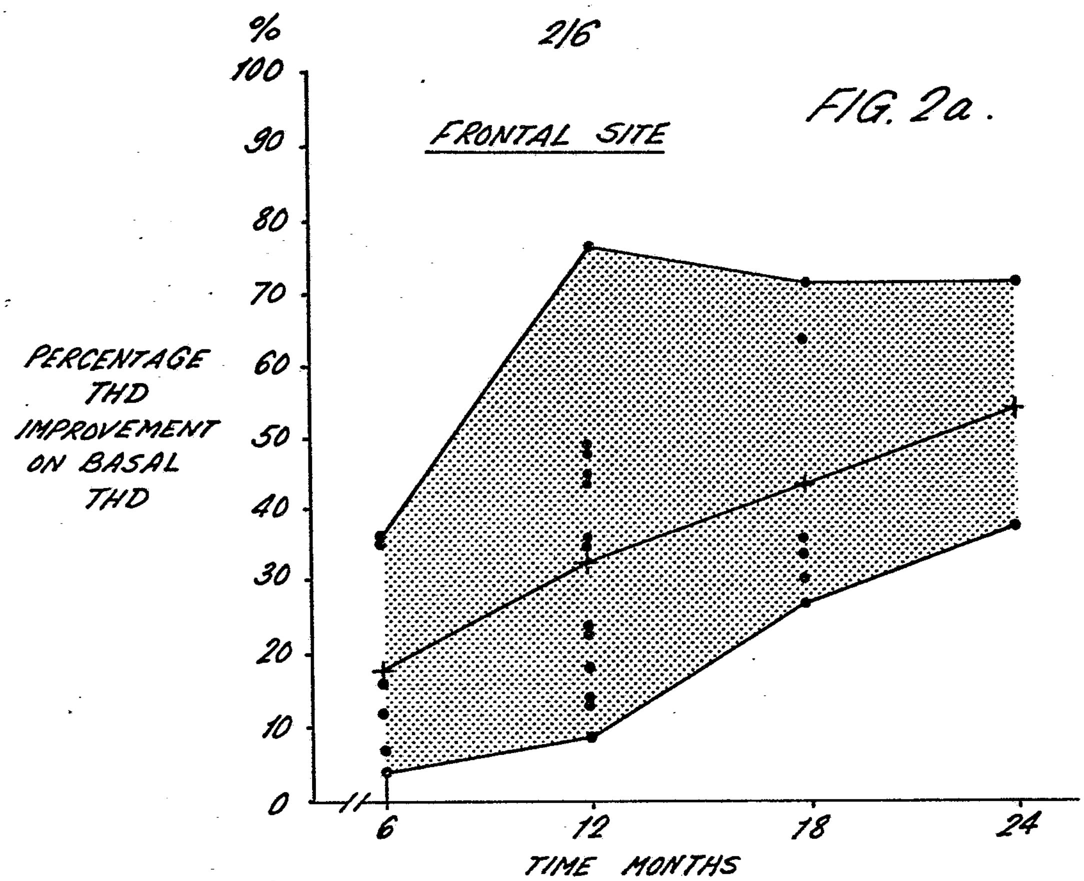
%
100
90
80
70
60
50
40
30
20
10
0
FRONTAL SITE
PERCENTAGE
THD
IMPROVEMENT
ON BASAL
THD
6
12
18
24
TIME MONTHS

FIG. 2a.

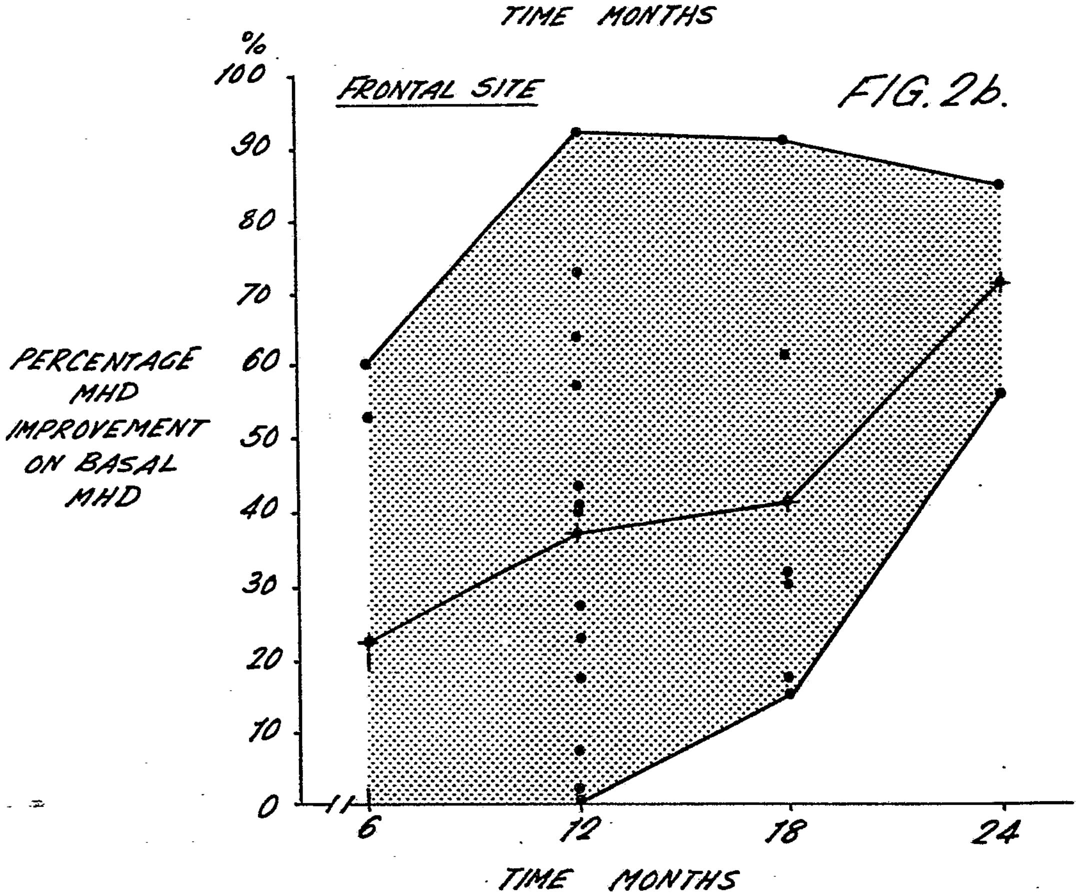
%
100
90
80
70
60
50
40
30
20
10
0
FRONTAL SITE
PERCENTAGE
MHD
IMPROVEMENT
ON BASAL
MHD
6
12
18
24
TIME MONTHS

FIG. 2b.

0163490

3/6

FIG. 3.

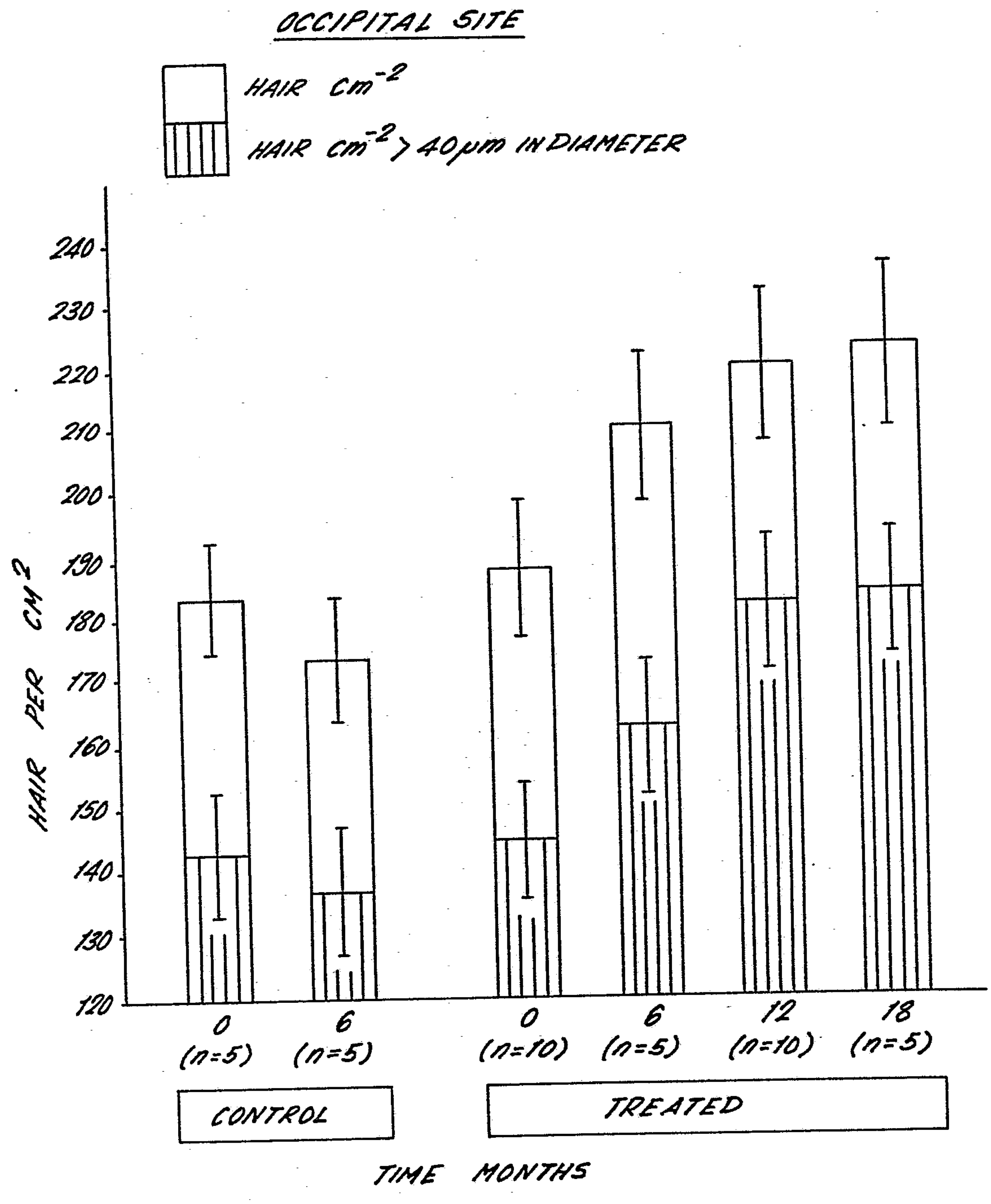

FIG. 4a.

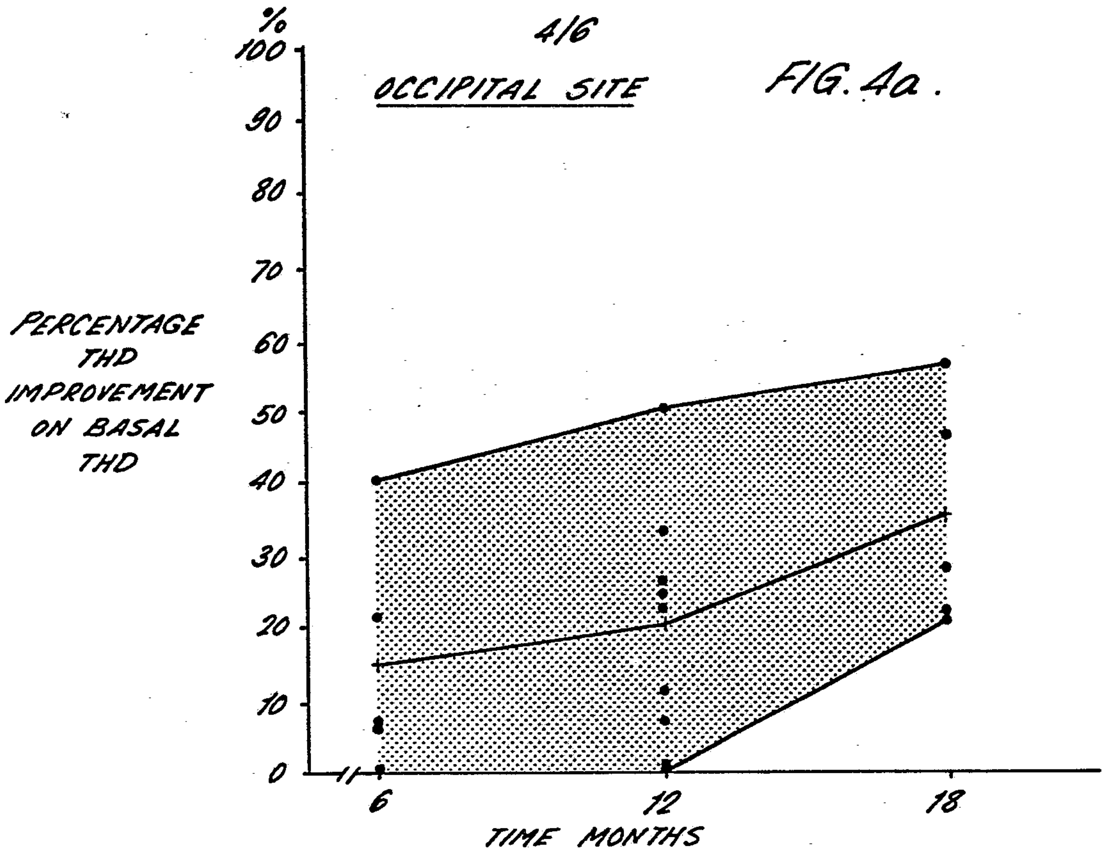
%
100
90
80
70
60
50
40
30
20
10
0
OCCIPITAL SITE
PERCENTAGE THD IMPROVEMENT ON BASAL THD
6
12
18
TIME MONTHS

FIG. 4b.

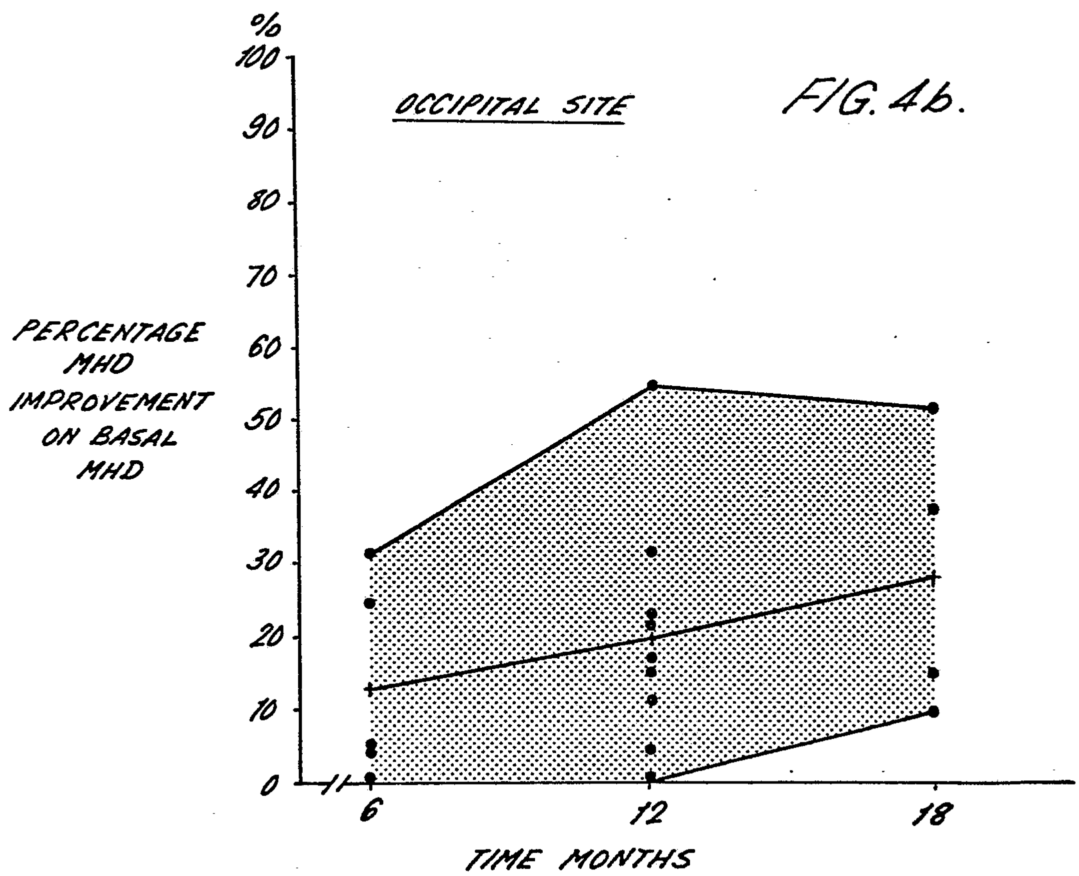
%
100
90
80
70
60
50
40
30
20
10
0
OCCIPITAL SITE
PERCENTAGE MHD IMPROVEMENT ON BASAL MHD
6
12
18
TIME MONTHS

5/6

FIG. 5.

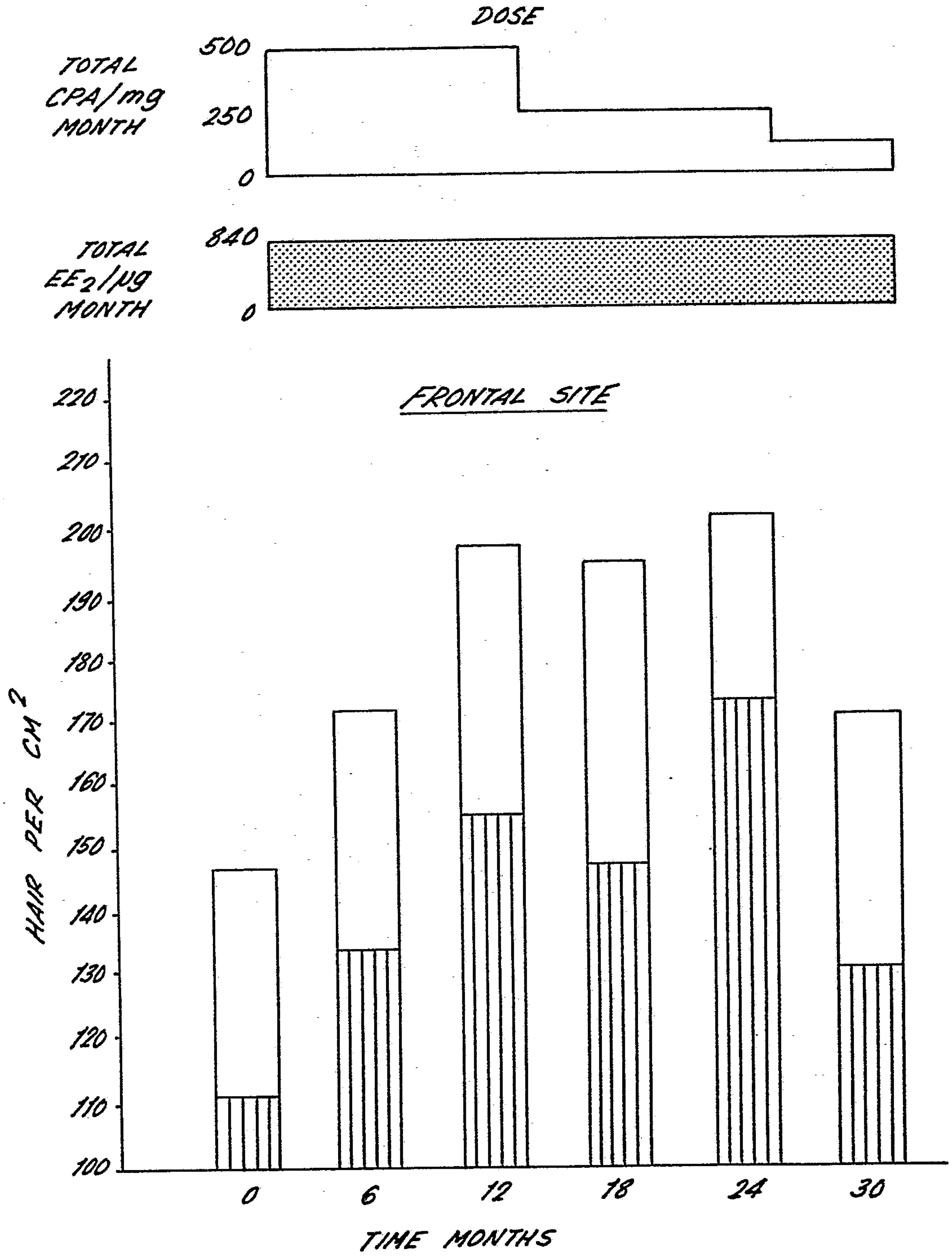
DOSE
TOTAL CPA/mg MONTH
500
250
0
TOTAL EE2/µg MONTH
840
0
FRONTAL SITE
HAIR PER CM2
220
210
200
190
180
170
160
150
140
130
120
110
100
0
6
12
18
24
30
TIME MONTHS

FIG. 6.

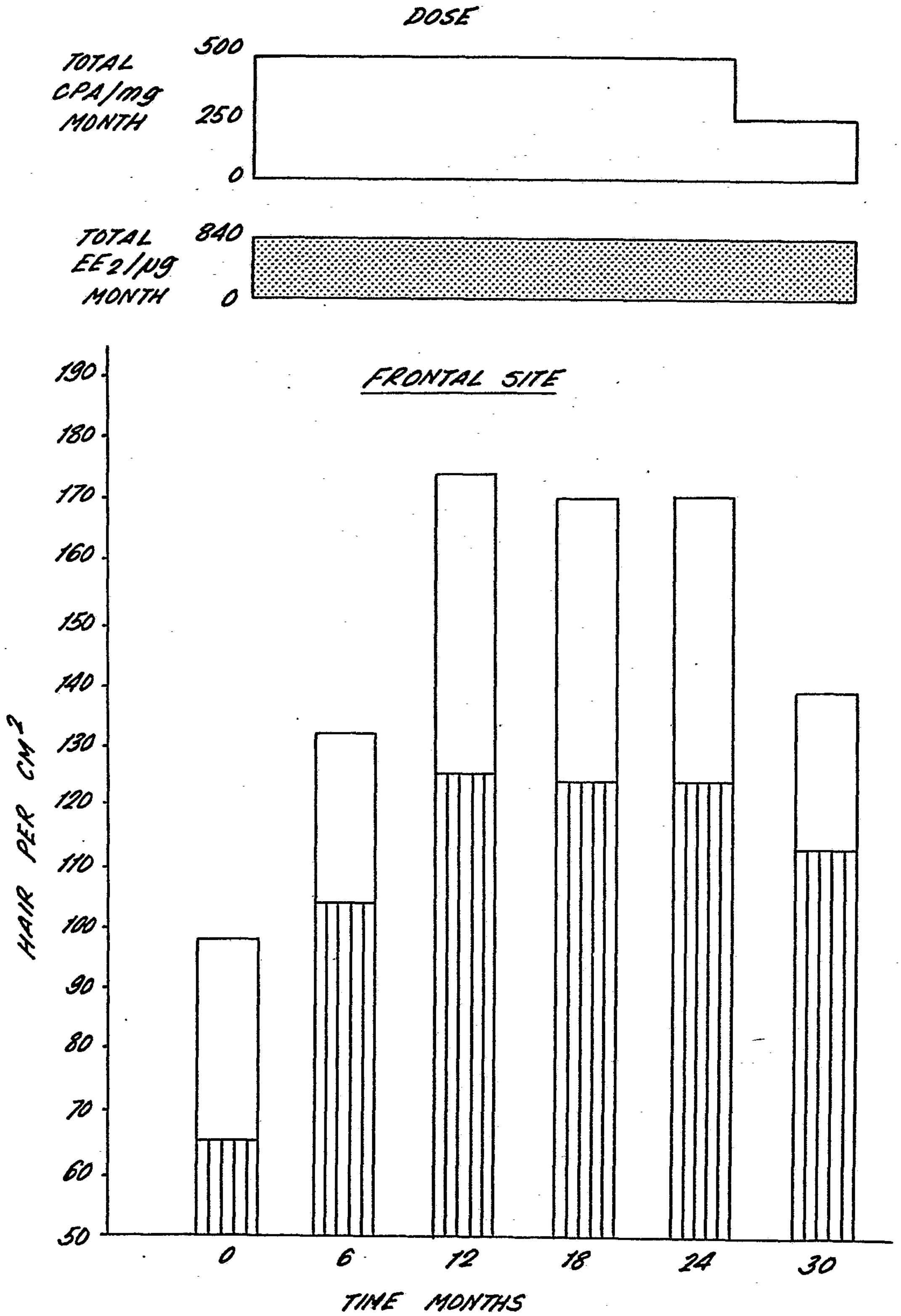
DOSE
TOTAL CPA/mg MONTH
500
250
0
TOTAL EE2/µg MONTH
840
0
FRONTAL SITE
HAIR PER CM²
190
180
170
160
150
140
130
120
110
100
90
80
70
60
50
0
6
12
18
24
30
TIME MONTHS

European Patent Office

# EUROPEAN SEARCH REPORT

0163490
Application number

EP 85 30 3624

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | ROTE LISTE, 1979, Editio Cantor, Aulendorf/Württ., DE; * Nr. 75 038B, "Diane" * | 1-10 | A 61 K 7/06<br>A 61 K 31/57 //<br>(A 61 K 31/57<br>A 61 K 31:565) |
| X | ULISTED DRUGS, vol. 26, no. 6, June 1974, page 83, point j, Chatham, N.J., US; * Page 83, point j, "SH 81041" * | 1-10 | |
| X | EP-A-0 033 164 (IRMGARD VON KISTOWSKI) * Page 5, claims 1-2 * | 1-10 | |
| A | DE-A-2 431 694 (ASCHE AG) * Pages 21-22, claims 1-9 * | 1-10 | TECHNICAL FIELDS SEARCHED (Int. Cl.4) |
| A | CHEMICAL ABSTRACTS, vol. 99, no. 19, 7th November 1983, page 78, no. 152385m, Columbus, Ohio, US; F.J.G. EBLING et al.: "Steroid inhibitors of androgen-potentiated actions on skin" & J. STEROID BIOCHEM: 1983, 19(1B), 587-90 * Abstract * | 1-10 | A 61 K |

The present search report has been drawn up for all claims

| Place of search | Date of completion of the search | Examiner |
|---|---|---|
| THE HAGUE | 21-08-1985 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82